# EUROPEAN PATENT APPLICATION

(11) **EP 3 248 997 A1**
(43) Date of publication of application: **29.11.2017**
(21) Application number: 17180083.2
(22) Date of filing: 29.06.2016
(51) Int. Cl.: C08G 61/10, C08G 61/12, C09D 165/00, C09D 165/02, C08L 65/00, C08L 65/02, H01B 3/30, H01L 23/532, H01L 51/00

(54) **POLYARYLENE MATERIALS**

(30) Priority: 06.07.2015 US 201562188943 P; 29.02.2016 US 201615056352
(62) Divisional of application: 16176893.2
(71) Applicant: Rohm and Haas Electronic Materials LLC, Marlborough, MA 01752 (US)
(72) Inventor: DING, Ping, Marlborough, MA Massachusetts 01752 (US); CHUANG, Peng-Wei, Santa Clara, CA California 95051 (US); GILMORE, Christopher, Marlborough, MA Massachusetts 01752 (US); KIARIE, Cecilia W., Marlborough, MA Massachusetts 01752 (US); KIM, Young-Seok, Marlborough, MA Massachusetts 01752 (US); ZHANG, Jieqian, Marlborough, MA Massachusetts 01752 (US); RACHFORD, Aaron A., Marlborough, MA Massachusetts 01752 (US); YAMADA, Shintaro, Marlborough, MA Massachusetts 01752 (US); CAMERON, James, Marlborough, MA Massachusetts 01752 (US)
(74) Representative: McLean, Craig Sutherland

(57) **Abstract**

Polyarylene oligomers formed from an aromatic dialkyne monomer having a solubility enhancing moiety show improved solubility in certain organic solvents and are useful in forming dielectric material layers in electronics applications.

## Description

This application claims the benefit of U.S. provisional application no. 62/188,943, filed on July 6, 2015.

The present invention relates generally to the field of polyarylene materials and more particularly to polyarylene oligomers for use in electronics applications.

Polymer dielectrics may be used as insulating layers in various electronic devices, such as integrated circuits, multichip modules, laminated circuit boards, displays and the like. The electronics fabrication industry has different requirements for dielectric materials, such as dielectric constant, coefficient of thermal expansion, modulus, and the like, depending upon the particular application.

Various inorganic materials, such as silica, silicon nitride and alumina, have been used as dielectric materials in electronic devices. These inorganic materials generally can be deposited in thin layers, typically by vapor deposition techniques, and have advantageous properties, such as not readily absorbing water. Polymer dielectric materials often possess properties which offer advantages over inorganic dielectric materials in certain applications, such as ease of application such as by spin-coating techniques, gap-filling ability, lower dielectric constants, and the ability to withstand certain stresses without fracturing, that is, polymer dielectrics can be less brittle than inorganic dielectric materials. However, polymer dielectrics often present challenges to process integration during fabrication. For example, to replace silicon dioxide as a dielectric in certain applications such as integrated circuits, the polymer dielectric must be able to withstand processing temperatures during metallization and annealing steps of the process. In general, the polymer dielectric material should have a glass transition temperature greater than the processing temperature of subsequent manufacturing steps. Also, the polymer dielectric should not absorb water which may cause an increase in the dielectric constant and potential corrosion of metal conductors.

Polyarylene polymers are well-known as dielectric materials and possess many desirable properties. For example, International Pat. App. No. WO 97/10193 discloses certain polyarylene oligomers prepared from certain ethynyl-substituted aromatic compounds and a biscyclopentadienone monomer. The aromatic ring in these ethynyl-substituted aromatic compounds may be substituted with certain substituents such as CF₃-, CF₃O-, ArO-, ArS-, or (Ar)₂P(=O)-, wherein Ar designates a certain aromatic ring. Polyarylene oligomers are prepared at relatively high temperatures in organic solvents having relatively high boiling points (typically ≥ 150 °C). However, such reaction solvents are poor choices as casting solvents in the electronics industry, and the polyarylene oligomers must be precipitated from the reaction solvent and taken up in a different organic solvent with a much lower boiling point that is suitable for casting films of these polymers. Such polyarylene oligomers suffer from limited solubility in organic solvents conventionally used in the electronics industry, limiting the use of these polymers. U.S. Pat. App. Serial No. 14/472,429 (Gilmore et al.), filed on August 29, 2014, discloses polar moiety-terminated polyarylene oligomers having improved solubility prepared by reacting a first monomer comprising two cyclopentadienone moieties, an ethynyl-substituted aromatic compound as a second monomer, and as a third monomer a monoethynyl-substituted compound of the formula 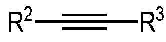 wherein R² is H, optionally substituted C₁₋₁₀ alkyl, optionally substituted C₇₋₁₂ aralkyl, optionally substituted C₆₋₁₀ aryl, or R³, and R³ is a polar moiety. While these polar moiety-terminated polyarylene oligomers do have improved solubility in certain organic solvents as compared to conventional polyarylene oligomers, the solubility improvement in some solvents is not sufficient to allow these polyarylene oligomers to be used in certain applications in the electronics industry. There remains a need in the industry for polyarylene polymers having improved solubility in organic solvents, particularly in organic solvents used to cast polymer films in the electronics industry.

The present invention provides a polyarylene polymer comprising as polymerized units one or more first monomers of formula (1): 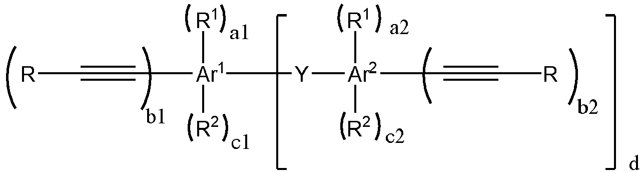 wherein each Ar¹ and Ar² is independently a C₆₋₃₀ aryl moiety; each R is independently chosen from H, C₆₋₃₀ aryl, and substituted C₆₋₃₀ aryl; each R¹ is independently chosen from -OH, C₁₋₆ hydroxyalkyl, -C(=O)OR³, -C(=O)N(R⁴)₂, -O-C(=O)R⁵, -NR⁴C(=O)R⁶, -N(R⁴)₃⁺ An⁻,-NO₂; -S(=O)₂-OR⁷, -O-S(=O)₂-R⁸, -NR⁴-S(=O)₂-R⁶, and S(=O)₂-N(R⁴)₂; each R² is independently chosen from C₁₋₁₀ alkyl, C₁₋₁₀ haloalkyl, C₁₋₁₀ hydroxyalkyl, C₁₋₁₀ alkoxy, CN, N(R⁴)₂, and halo; R³ = H, C₁₋₁₀ alkyl, C₁₋₁₀ hydroxyalkyl, C₁₋₁₀ aminoalkyl, C₆₋₃₀ aryl, or M; each R⁴ is independently H, C₆₋₃₀ aryl, or C₁₋₁₀ alkyl; each R⁵ is independently chosen from H, C₁₋₁₀ alkyl, C₁₋₁₀ hydroxyalkyl, C₆₋₃₀ aryl, -O(C₁₋₁₀ alkyl), -O(C₆₋₁₀ aryl) and -N(R⁴)₂; R⁶ = H, C₁₋₁₀ alkyl, C₁₋₁₀ hydroxyalkyl, C₆₋₃₀ aryl, -O(C₁₋₁₀ alkyl), or -NH(C₁₋₁₀ alkyl); R⁷ = H, C₁₋₁₀ alkyl, C₆₋₃₀ aryl, or M; R⁸ = C₆₋₃₀ aryl, C₁₋₁₀ alkyl, and halo C₁₋₁₀ alkyl; M = an alkali metal ion, an alkaline earth metal ion, or an ammonium ion; An⁻ is an anion chosen from halide and C₁₋₂₀ carboxylate; Y is a chemical bond or a divalent linking group chosen from - O-, -S-, -S(=O)-, -S(=O)₂-, -C(=O)-, -(C(R⁹)₂)_{z}-, C₆₋₃₀ aryl, and -(C(R⁹)₂)_{z1}-(C₆₋₃₀ aryl)-(C(R⁹)₂)_{z2}-; each R⁹ is independently chosen from H, hydroxy, halo, C₁₋₁₀ alkyl, C₁₋₁₀ haloalkyl, and C₆₋₃₀ aryl; a1 = 0 to 3; a2 = 0 to 3; b1 = 1 to 4; b2 = 0 to 2; c1 = 0 to 2; c2 = 0 to 2; a1 + a2 = 1 to 6; b1 + b2 = 2 to 6; c1 + c2 = 1 to 6; d = 0 to 2; z = 1 to 10; z1 = 0 to 10; z2 = 0 to 10; and z1 + z2 = 1 to 10; and one or more second monomers comprising two cyclopentadienone moieties.

Also provided by the present invention is a composition comprising one or more polyarylene polymers described above and one or more organic solvents.

Further, the present invention provides a method of forming a dielectric material layer comprising: disposing a layer of the composition described above on a substrate surface; removing the organic solvent; and curing the oligomer to form a dielectric material layer.

This invention still further provides a structure comprising a dielectric layer disposed on a surface of a substrate, wherein the dielectric layer is formed from the polyarylene polymer described above.

Still further, the present invention provides a monomer of formula (1): 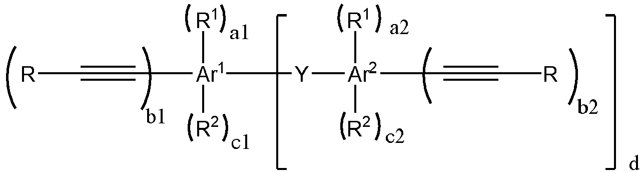 each Ar¹ and Ar² is independently a C₆₋₃₀ aryl moiety; each R is independently chosen from H, C₆₋₃₀ aryl, and substituted C₆₋₃₀ aryl; each R¹ is independently chosen from -OH, C₁₋₆ hydroxyalkyl, -C(=O)OR³, -C(=O)N(R⁴)₂, -O-C(=O)R⁵, -NR⁴C(=O)R⁶, -N(R⁴)₃⁺ An⁻, -NO₂; -S(=O)₂-OR⁷, -O-S(=O)₂-R⁸, -NR⁴-S(=O)₂-R⁶, and S(=O)₂-N(R⁴)₂; each R² is independently chosen from C₁₋₁₀ alkyl, C₁₋₁₀ haloalkyl, C₁₋₁₀ hydroxyalkyl, C₁₋₁₀ alkoxy, CN, N(R⁴)₂, and halo; R³ = H, C₁₋₁₀ alkyl, C₁₋₁₀ hydroxyalkyl, C₁₋₁₀ aminoalkyl, C₆₋₃₀ aryl, or M; each R⁴ is independently H, C₆₋₃₀ aryl, or C₁₋₁₀ alkyl; each R⁵ is independently chosen from H, C₁₋₁₀ alkyl, C₁₋₁₀ hydroxyalkyl, C₆₋₃₀ aryl, -O(C₁₋₁₀ alkyl), -O(C₆₋₁₀ aryl) and -N(R⁴)₂; R⁶ = H, C₁₋₁₀ alkyl, C₁₋₁₀ hydroxyalkyl, C₆₋₃₀ aryl,-O(C₁₋₁₀ alkyl), or -NH(C₁₋₁₀ alkyl); R⁷ = H, C₁₋₁₀ alkyl, C₆₋₃₀ aryl, or M; R⁸ = C₆₋₃₀ aryl, C₁₋₁₀ alkyl, and halo C₁₋₁₀ alkyl; M = an alkali metal ion, an alkaline earth metal ion, or an ammonium ion; An⁻ is an anion chosen from halide and C₁₋₂₀ carboxylate; Y is a chemical bond or a divalent linking group chosen from -O-, -S-, - S(=O)-, -S(=O)₂-, -C(=O)-, -(C(R⁹)₂)_{z}-, C₆₋₃₀ aryl, and -(C(R⁹)₂)_{z1}-(C₆₋₃₀ aryl)-(C(R⁹)₂)_{z2}-; each R⁹ is independently chosen from H, hydroxy, halo, C₁₋₁₀ alkyl, C₁₋₁₀ haloalkyl, and C₆₋₃₀ aryl; a1 = 0 to 3; a2 = 0 to 3; b1 = 1 to 4; b2 = 0 to 2; c1 = 0 to 2; c2 = 0 to 2; a1 + a2 = 1 to 6; b1 + b2 = 2 to 6; c1 + c2 = 1 to 6; d = 0 to 2; z = 1 to 10; z1 = 0 to 10; z2 = 0 to 10; and z1 + z2 = 1 to 10.

As used throughout this specification, the following abbreviations shall have the following meanings, unless the context clearly indicates otherwise: °C = degree Celsius; g = gram; mg = milligram; L = liter; mL = milliliter; Å = angstrom; nm = nanometer; µm = micron = micrometer; mm = millimeter; sec. = second; min. = minute; hr. = hour; DI = deionized; and Da = daltons. Unless otherwise specified, all amounts are percent by weight ("wt%") and all ratios are molar ratios. All numerical ranges are inclusive and combinable in any order, except where it is clear that such numerical ranges are constrained to add up to 100%. The articles "a", "an" and "the" refer to the singular and the plural. "Alkyl" refers to linear, branched and cyclic alkyl unless otherwise specified. "Alkyl" refers to an alkane radical, and includes alkane monoradicals, diradicals (alkylene), and higher-radicals. "Halo" refers to fluoro, chloro, bromo, and iodo. When an element is referred to as being "disposed on" another element, it can be directly on the other element or intervening elements may be present therebetween. In contrast, when an element is referred to as being "disposed directly on" another element, there are no intervening elements present.

The present invention relates to polyarylene polymers. "Aryl" refers to aromatic carbocycles and aromatic heterocycles. The term "aryl" refers to an aromatic radical, and includes monoradicals, diradicals (arylene), and higher-radicals. It is preferred that aryl moieties are aromatic carbocycles. "Substituted aryl" refers to any aryl moiety having one or more of its hydrogens replaced with one or more substituents chosen from halogen, C₁₋₆ alkyl, halo C₁₋₆ alkyl, C₁₋₆ alkoxy, halo C₁₋₆ alkoxy, phenyl, and phenoxy, preferably from halogen, C₁₋₆ alkyl, halo C₁₋₄ alkyl, C₁₋₆ alkoxy, halo C₁₋₄ alkoxy, and phenyl, and more preferably from halogen, C₁₋₆ alkyl, C₁₋₆ alkoxy, phenyl, and phenoxy. Preferably, a substituted aryl has from 1 to 3 substituents, and more preferably 1 or 2 substituents. As used herein, the term "polymer" includes oligomers. The term "oligomer" refers to dimers, trimers, tetramers and other polymeric materials that are capable of further curing. By the term "curing" is meant any process, such as polymerization or condensation, that increases the overall molecular weight of the present oligomers, removes solubility enhancing groups from the present oligomers, or both increases the overall molecular weight and removes solubility enhancing groups. "Curable" refers to any material capable of being cured under certain conditions.

Polymers of the present invention comprise polymerized units of one or more aromatic compounds having two or more ethynyl moieties and one or more solubility enhancing moieties as a first monomer and one or more compounds having two cyclopentadienone moieties as a second monomer. Preferably, the first monomers have two ethynyl moieties and one or more solubility enhancing moieties. The first monomers preferably have from 1 to 3 solubility enhancing moieties, and more preferably 1 or 2 solubility enhancing moieties. Optionally, the polymers of the invention may further include as polymerized units one or more third monomers, where the third monomer is an aromatic compound having two or more ethynyl moieties and being free of solubility enhancing moieties. In still another option, the present polymers may comprise as polymerized units one or more end capping monomers. Preferred polymers of the present invention are oligomers.

The one or more first monomers of the present polymers have the formula (1): 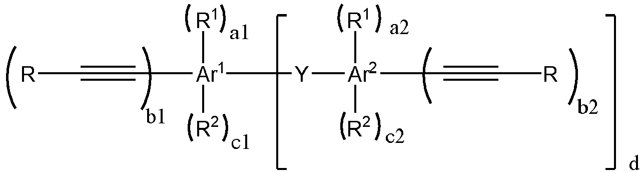 each Ar¹ and Ar² is independently a C₆₋₃₀ aryl moiety; each R is independently chosen from H, C₆₋₃₀ aryl, and substituted C₆₋₃₀ aryl; each R¹ is independently chosen from -OH, C₁₋₆ hydroxyalkyl, -C(=O)OR³, -C(=O)N(R⁴)₂, -O-C(=O)R⁵, -NR⁴C(=O)R⁶, -N(R⁴)₃⁺ An⁻, -NO₂; -S(=O)₂-OR⁷, -O-S(=O)₂-R⁸, -NR⁴-S(=O)₂-R⁶, and S(=O)₂-N(R⁴)₂; each R² is independently chosen from C₁₋₁₀ alkyl, C₁₋₁₀ haloalkyl, C₁₋₁₀ hydroxyalkyl, C₁₋₁₀ alkoxy, CN, N(R⁴)₂, and halo; R³ = H, C₁₋₁₀ alkyl, C₁₋₁₀ hydroxyalkyl, C₁₋₁₀ aminoalkyl, C₆₋₃₀ aryl, or M; each R⁴ is independently H, C₆₋₃₀ aryl, or C₁₋₁₀ alkyl; each R⁵ is independently chosen from H, C₁₋₁₀ alkyl, C₁₋₁₀ hydroxyalkyl, C₆₋₃₀ aryl, -O(C₁₋₁₀ alkyl), -O(C₆₋₁₀ aryl) and -N(R⁴)₂; R⁶ = H, C₁₋₁₀ alkyl, C₁₋₁₀ hydroxyalkyl, C₆₋₃₀ aryl, -O(C₁₋₁₀ alkyl), or -NH(C₁₋₁₀ alkyl); R⁷ = H, C₁₋₁₀ alkyl, C₆₋₃₀ aryl, or M; R⁸ = C₆₋₃₀ aryl, C₁₋₁₀ alkyl, and halo C₁₋₁₀ alkyl; M = an alkali metal ion, an alkaline earth metal ion, or an ammonium ion; An⁻ is an anion chosen from halide and C₁₋₂₀ carboxylate; Y is a chemical bond or a divalent linking group chosen from -O-, -S-,-S(=O)-, -S(=O)₂-, -C(=O)-, -(C(R⁹)₂)_{z}-, C₆₋₃₀ aryl, and -(C(R⁹)₂)_{z1}-(C₆₋₃₀ aryl)-(C(R⁹)₂)_{z2}-; each R⁹ is independently chosen from H, hydroxy, halo, C₁₋₁₀ alkyl, C₁₋₁₀ haloalkyl, and C₆₋₃₀ aryl; a1 = 0 to 3; a2 = 0 to 3; b1 = 1 to 4; b2 = 0 to 2; c1 = 0 to 2; c2 = 0 to 2; a1 + a2 = 1 to 6; b1 + b2 = 2 to 6; c1 + c2 = 1 to 6; d = 0 to 2; z = 1 to 10; z1 = 0 to 10; z2 = 0 to 10; and z1 + z2 = 1 to 10. Each R is preferably independently chosen from H and C₆₋₂₀ aryl, more preferably from H and C₆₋₁₀ aryl, and yet more preferably from H and phenyl. It is preferred that each R¹ is independently chosen from -OH, C₁₋₄ hydroxyalkyl, -C(=O)OR³,-C(=O)N(R⁴)₂, -O-C(=O)R⁵, -S(=O)₂-OR⁶, and S(=O)₂-N(R⁴)_{2'} more preferably from -OH, C₁₋₄ hydroxyalkyl, -C(=O)OR³ and -C(=O)N(R⁴)₂, and yet more preferably -OH and - C(=O)OR³. It is preferred that each R² is independently chosen from C₁₋₁₀ alkyl, C₁₋₁₀ haloalkyl, C₁₋₁₀ hydroxyalkyl, C₁₋₁₀ alkoxy, N(R⁴)₂, and halo, and more preferably from C₁₋₁₀ alkyl, C₁₋₁₀ haloalkyl, and halo. Preferably, R³ is H, C₁₋₆ alkyl, C₁₋₆ hydroxyalkyl, C₁₋₆ aminoalkyl, C₆₋₃₀ aryl, or M, more preferably H, C₁₋₄ alkyl, C₁₋₆ hydroxyalkyl, or M, and even more preferably H or M. R⁴ is preferably H, C₆₋₃₀ aryl, or C₁₋₆ alkyl, and more preferably H or C₁₋₄ alkyl. It is preferred that R⁵ is C₁₋₆ alkyl, C₁₋₆ hydroxyalkyl, C₆₋₃₀ aryl, -O(C₁₋₁₀ alkyl), or -N(R⁴)₂, and more preferably C₁₋₆ alkyl, C₁₋₆ hydroxyalkyl, C₆₋₂₀ aryl, -O(C₁₋₆ alkyl), or - N(R⁴)₂. R⁶ is preferably H, C₁₋₁₀ alkyl, C₁₋₆ hydroxyalkyl, C₆₋₂₀ aryl, -O(C₁₋₁₀ alkyl), or - N(R⁴)₂, and more preferably H, C₁₋₆ alkyl, -O(C₁₋₆ alkyl), or -N(R⁴)₂. R⁷ is preferably H, C₁₋₆ alkyl, C₆₋₂₀ aryl, or M, more preferably H, C₁₋₄ alkyl, or M, and even more preferably H or M. Preferably, Y is a chemical bond or a divalent linking group chosen from -O-, -S-, - S(=O)-, -S(=O)₂-, -C(=O)-, -(C(R⁹)₂)_{z}-, and C₆₋₃₀ aryl, and more preferably a chemical bond, -O-, -S-, -S(=O)₂-, -C(=O)-, and -(C(R⁹)₂)_{z}-. It is preferred that R⁸ is C₆₋₂₀ aryl, C₁₋₁₀ alkyl, and C₁₋₁₀ fluoroalkyl, and more preferably phenyl, tolyl, methyl, and trifluoromethyl. R⁹ is preferably H, halo, C₁₋₁₀ alkyl, halo C₁₋₁₀ alkyl, and C₆₋₃₀ aryl, and more preferably fluoro, C₁₋₆ alkyl, fluoro C₁₋₆ alkyl, and C₆₋₂₀ aryl. Any suitable ammonium ion may be used for M, such as those of the formula [NA₄]⁺ wherein each A is independently chosen from H, C₁₋₆ alkyl, C₇₋₁₀ aralkyl and C₆₋₈ aryl. Exemplary ammonium ions include, without limitation, ammonium, tetramethylammonium, tetraethylammonium, tetrabenzylammonium, and tetraphenylammonium. Preferred alkali metal ions for M are lithium ions, sodium ions or potassium ions. Preferred alkaline earth ions for M are magnesium ions or calcium ions. M is preferably chosen from lithium ions, sodium ions, potassium ions, magnesium ions, calcium ions and ammonium ions, more preferably from lithium ions, sodium ions, potassium ions, and ammonium ions, and even more preferably from lithium ions, sodium ions, potassium ions, and ammonium ions of the formula [NA₄]⁺ wherein each A is independently chosen from H, C₁₋₆ alkyl, C₇₋₁₀ aralkyl and C₆₋₈ aryl. More preferably, M is chosen from alkali metal ions and ammonium ions. An- is preferably chosen from halide and C₁₋₁₀ carboxylate, and more preferably halide and C₁₋₆ carboxylate. Preferably, a1 = 1 to 3, more preferably 1 to 2, and most preferably a1 = 1. It is preferred that a2 = 0 to 2. Preferably, a1 + a2 = 1 to 4, more preferably 1 to 3, and yet more preferably 1 to 2. It is preferred that b1 = 1 to 2, and more preferably 2. It is preferred that b2 = 0 or 1. Preferably, b1 + b2 = 2 to 4, and more preferably 2 or 3, and even more preferably 2. Preferably c1 = 0 or 1, and more preferably 0. C2 is preferably 0 or 1, and more preferably 0. C1 + c2 is preferably 0 to 3, more preferably 0 to 2, and even more preferably 0. It is preferred that d = 0 or 1, and more preferably 0. Preferably, z = 1 to 6, more preferably 1 to 3, and even more preferably z = 1. Z1 and z2 are each preferably 0 to 5. Preferably z1 + z2 = 1 to 6, and more preferably 2 to 6.

Suitable aryl moieties for Ar¹ and Ar² include, but are not limited to, phenyl, naphthyl, anthracenyl, phenanthryl, pyrenyl, coronenyl, tetracenyl, pentacenyl, tetraphenyl, benzotetracenyl, triphenylenyl, and perylenyl. It is preferred that Ar¹ and Ar² in formula (1) are independently a C₆₋₂₀ aryl moiety. Preferred aryl moieties for Ar¹ and Ar² are phenyl, naphthyl, anthracenyl, phenanthryl, pyrenyl, tetracenyl, pentacenyl, tetraphenyl, triphenylenyl, and perylenyl.

Preferred first monomers are those of formulas (2) and (3): 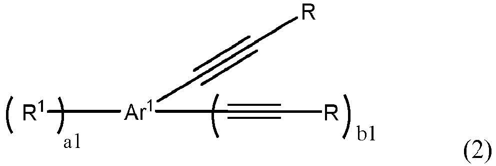 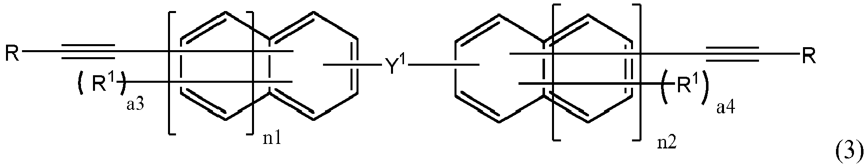 wherein Ar¹, R, R¹, a1 and b1 are as defined above; a3 is 1 or 2; a4 is 0 to 2; each of n1 and n2 is independently 0 to 4; and Y¹ is a chemical bond, O, S, S(=O)₂, C(=O), C(CH₃)₂, CF₂, and C(CF₃)₂. It will be appreciated by those skilled in the art that the brackets ("[ ]") in formula (3) refer to the number of aromatic rings fused to the phenyl ring. Accordingly, when nl (or n2) = 0, the aromatic moiety is phenyl; when nl (or n2) = 1, the aromatic moiety is naphthyl; when nl (or n2) = 2, the aromatic moiety may be anthracenyl or phenanthryl; when nl (or n2) = 3, the aromatic moiety may be tretacenyl, tetraphenyl, triphenylenyl, or pyrenyl; and when nl (or n2) = 4, the aromatic moiety may be perylenyl or benzotetracenyl. In formula (2), a1 is preferably 1 to 2, and more preferably a1 = 1. It is preferred that b1 in formula (2) is 1 or 2, and more preferably 1. R is preferably H or phenyl. R¹ in each of formulas (2) and (3) is preferably -OH, C₁₋₆ hydroxyalkyl, -C(=O)OR³, -C(=O)N(R⁴)₂, -O-C(=O)R⁵,-S(=O)₂-OR⁶, and S(=O)₂-N(R⁴)₂, more preferably from -OH, C₁₋₄ hydroxyalkyl, -C(=O)OR³ and -C(=O)N(R⁴)₂, and yet more preferably -OH and -C(=O)OR³. Ar¹ in formula (2) is preferably phenyl, naphthyl, anthracenyl, pyrenyl, and perylenyl. In formula (3), it is preferred that n1 and n2 are independently chosen from 0, 1, 3, and 4, more preferably from 0, 1 and 3, and even more preferably from 1 and 3. It is further preferred that nl = n2. In formula (3), Y¹ is preferably a chemical bond, O, S(=O)₂, C(=O), C(CH₃)₂, CF₂, and C(CF₃)₂, and more preferably a chemical bond.

Particularly preferred monomers of formula (2) are monomers of formulas (4) to (8): 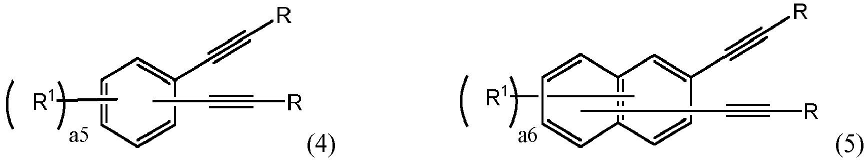 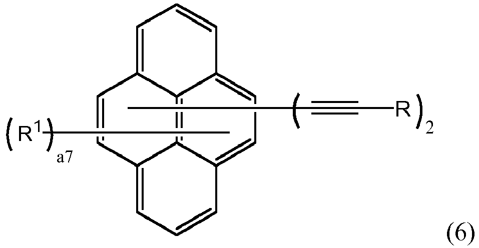 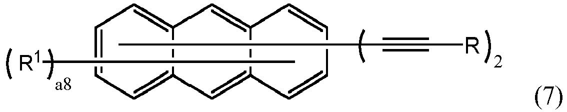 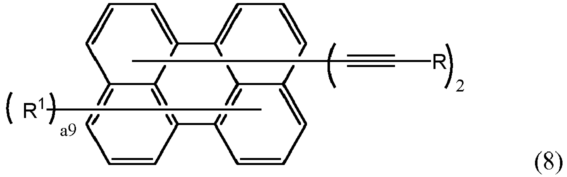 wherein R and R¹ are as described above; a5 = 1 or 2; and each of a6, a7, a8 and a9 are independently 1 to 4. Preferably, a5 = 1. It is preferred that a6 is 1 to 3, more preferably 1 or 2, and even more preferably 1. Preferably, each of a7 to a9 is independently 1 to 3, and more preferably 1 to 2.

In the monomers of formula (1), any two alkynyl moieties may have an ortho, meta or para relationship to each other, and preferably a meta or para relationship to each other. Preferably, the alkynyl moieties in the monomers of formula (1) do not have an ortho relationship to each other. Suitable monomers of formula (1) are generally commercially available or may be readily prepared by methods known in the art.

The present polyarylene polymers may be comprised of one monomer of formula (1), or a mixture of two or more monomers of formula (1). Monomers of formula (2) are preferred first monomers. It is preferred that the present polymers are comprised of polymerized units of one or more monomers of formula (2). In an alternate preferred embodiment, the present polymers are comprised of polymerized units of one or more monomers of formula (3), or in yet another alternate embodiment of one or more monomers of formula (2) and one or more monomers of formula (3). Mixtures of polymers comprising as polymerized units one or more monomers of formula (1) may suitably be used.

Any monomer containing two cyclopentadienone moieties may suitably be used as the second monomer to prepare the present polymers. Alternatively, a mixture of 2 or more different monomers, each having two cyclopentadienone moieties, may be used as the second monomer. Such monomers containing two cyclopentadienone moieties are well-known in the art, such as those described in U.S. Pat. Nos. 5,965,679; 6,288,188; and 6,646,081; and in Int. Pat. Pubs. WO 97/10193 and WO 2004/073824. It is preferred that the second monomer has the structure shown in formula (9) 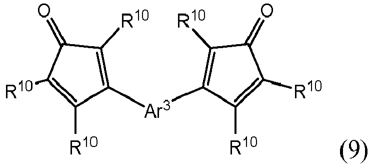 wherein each R¹⁰ is independently chosen from H, C₁₋₆ alkyl, or optionally substituted aryl; and Ar³ is an aromatic moiety. Preferably, each R¹⁰ is independently chosen from C₃₋₆ alkyl, phenyl and substituted phenyl, and more preferably each R¹⁰ is phenyl. A wide variety of aromatic moieties are suitable for use as Ar³, such as those disclosed in U.S. Pat. No. 5,965,679. Exemplary aromatic moieties useful for Ar³ include those having the structure shown in formula (10)

-(-Ar⁴-)ₓ-(-z-Ar⁴-)_{y}- (10)

wherein x is an integer chosen from 1, 2 or 3; y is an integer chosen from 0, 1, or 2; each Ar⁴ is independently chosen from 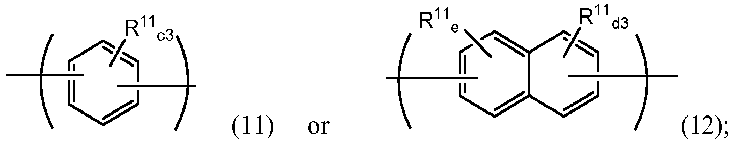 each R¹¹ is independently chosen from halogen, C₁₋₆ alkyl, halo C₁₋₆ alkyl, C₁₋₆ alkoxy, halo C₁₋₆ alkoxy, phenyl, and phenoxy; c3 is an integer from 0 to 4; each of d3 and e is an integer from 0 to 3; each Z is independently chosen from O, S, NR¹², PR¹², P(=O)R¹², C(=O), CR¹³R¹⁴, and SiR¹³R¹⁴; R¹², R¹³, and R¹⁴ are independently chosen from H, C₁₋₄ alkyl, halo C₁₋₄ alkyl, and phenyl. It is preferred that x is 1 or 2, and more preferably 1. It is preferred that y is 0 or 1, and more preferably 1. Preferably, each R¹¹ is independently chosen from halogen, C₁₋₄ alkyl, halo C₁₋₄ alkyl, C₁₋₄ alkoxy, halo C₁₋₄ alkoxy, and phenyl, and more preferably from fluoro, C₁₋₄ alkyl, fluoro C₁₋₄ alkyl, C₁₋₄ alkoxy, fluoro C₁₋₄ alkoxy, and phenyl. It is preferred that c3 is from 0 to 3, more preferably from 0 to 2, and yet more preferably 0 or 1. It is preferred that each of d3 and e is independently 0 to 2, and more preferably 0 or 1. In formula (12), it is preferred that d3 + e = 0 to 4, and more preferably 0 to 2. Each Z is preferably independently chosen from O, S, NR¹², C(=O), CR¹³R¹⁴, and SiR¹³R¹⁴, more preferably from O, S, C(=O), and CR¹³R¹⁴, and yet more preferably from O, C(=O), and CR¹³R¹⁴. It is preferred that each R¹², R¹³, and R¹⁴ are independently chosen from H, C₁₋₄ alkyl, fluoro C₁₋₄ alkyl, and phenyl; and more preferably from H, C₁₋₄ alkyl, fluoro C₁₋₂ alkyl, and phenyl. Preferably, each Ar⁴ has the formula (11).

One or more optional third monomers which may be used to form the present polymers are those of formula (13) 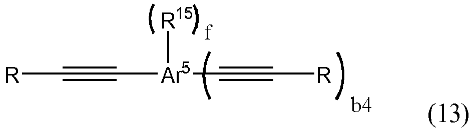 wherein each R is as defined above for the monomers of formula (1); Ar⁵ is a C₆₋₃₀ aromatic moiety; each R¹⁵ is independently chosen from C₁₋₄ alkyl, C₁₋₄ haloalkyl, C₁₋₄ alkoxy, optionally substituted C₇₋₁₄ aralkyl, and optionally substituted C₆₋₁₀ aryl; b4 = 1 or 2; and f = 0 to 4. "Substituted aralkyl" refers to an aralkyl moiety having one or more of its hydrogens replaced with one or more substituents chosen from halogen, C₁₋₆ alkyl, C₁₋₆ haloalkyl, C₁₋₆ alkoxy, C₁₋₆ haloalkoxy, phenyl, and phenoxy, preferably from halogen, C₁₋₆ alkyl, C₁₋₄ haloalkyl, C₁₋₆ alkoxy, C₁₋₄ haloalkoxy, and phenyl, and more preferably from halogen, C₁₋₆ alkyl, C₁₋₆ alkoxy, phenyl, and phenoxy. Fluorine is the preferred halogen. In formula (13), it is preferred that each R is independently H or C₆₋₁₀ aryl, and more preferably H or phenyl. It is preferred that each R¹⁵ is independently chosen from C₁₋₄ alkyl, C₁₋₄ fluoroalkyl, C₁₋₄ alkoxy, benzyl, phenethyl, phenyl, naphthyl, substituted phenyl and substituted naphthyl, more preferably C₁₋₂ alkyl, C₁₋₄ fluoroalkyl, C₁₋₂ alkoxy, phenyl, and substituted phenyl, and yet more preferably from C₁₋₂ alkyl, C₁₋₄ fluoroalkyl, C₁₋₂ alkoxy, and phenyl. Preferably, b4 = 2. Preferably, f = 0 to 3, more preferably 0 to 2, and yet more preferably f = 0. Ar5 may be any suitable C6-30 aromatic moiety, such as, without limitation, phenyl, naphthyl, anthracenyl, phenanthryl, tetracenyl, pyrenyl, perylenyl, coronenyl, pentacenyl, triphenylenyl, tetraphenyl, benzotetracenyl, biphenyl, binaphthyl, diphenyl ether, and dinaphthyl ether. Preferably, optional monomers of formula (13) comprise 2 or 3 alkynyl moieties having a terminal hydrogen or terminal phenyl moiety. Any 2 alkynyl moieties in the monomers of formula (13) may have an ortho, meta or para relationship to each other, and preferably a meta or para relationship to each other. Preferably, the alkynyl moieties do not have an ortho relationship to each other. A single optional monomer of formula (13) may be used to prepare the present polymers, or two or more optional monomers of formula (13) but different from each other may be used. When a single optional monomer of formula (13) is used, it is preferred that b4 = 2. In one preferred embodiment, the present polymers further comprise as polymerized units a monomer of formula (13), and more preferably a monomer of formula (13) wherein b4 = 2. In an alternate preferred embodiment, the present polymers further comprise as polymerized units one monomer of formula (13) wherein b4 = 1, and another monomer of formula (13) wherein b4 = 2.

Compounds useful as the third monomers of formula (13) are generally commercially available, or may be prepared by methods known in the art. Preferred optional monomers of formula (13) are: 1,3-diethynylbenzene; 1,4-diethynylbenzene; 4,4'-diethynyl-1,1'-diphenyl; 3,5-diethynyl-1,1'-biphenyl; 1,3,5-triethynylbenzene; 1,3-diethynyl-5-(phenylethynyl)benzene; 1,3-bis(phenylethynyl)benzene; 1,4-bis(phenylethynyl)benzene; 1,3,5-tris(phenylethynyl)benzene; 4,4'-bis(phenylethynyl)-1,1'-biphenyl; 4,4'-diethynyl-diphenylether; and mixtures thereof. More preferably, the monomers of formula (7) are chosen from: 1,3-diethynylbenzene; 1,4-diethynylbenzene; 1,3,5-triethynylbenzene; 4,4'-diethynyl-1,1'-biphenyl; 1,3-bis(phenylethynyl)-benzene; 1,4-bis(phenylethynyl)benzene; 4,4'-bis(phenylethynyl)-1,1'-biphenyl; and mixtures thereof. Even more preferably, the second monomers are chosen from: 1,3-diethynylbenzene; 1,4-diethynylbenzene; 4,4'-diethynyl-1,1'-biphenyl; 1,3,5-triethynylbenzene; and mixtures thereof.

Optionally, one or more end capping monomers may be used to prepare the present polyarylene polymers. Such end capping monomers have a single alkyne moiety and a solubility improving polar group and which function to cap one end, preferably two ends, and more preferably all ends, of the present polymers. Suitable monomers are those disclosed in U.S. Pat. App. Serial No. 14/472,429 (Gilmore et al.). It will be appreciated by those skilled in the art that reaction conditions can be selected such that these optional end capping monomers preferentially react with alkynyl moieties having terminal hydrogens (R = H) in the polymer over alkynyl moieties having terminal aryl moieties (R = C₆₋₂₀ aryl). Preferably, the polar moieties present in these optional end capping monomers are cleavable under conditions used to cure the present polyarylene polymers. Suitable optional end capping monomers are those of formula (14): 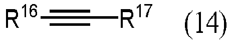 wherein R¹⁶ is H, optionally substituted C₁₋₁₀ alkyl, optionally substituted C₇₋₁₂ aralkyl, optionally substituted C₆₋₁₀ aryl, or R¹⁷; and R¹⁷ is a polar moiety. Suitable polar moieties are any hydrocarbyl moiety having from 1 to 20 carbon atoms and one or more functional groups chosen from -C(=O)-R¹⁸, -C(=O)OR¹⁸, -OH, -NO₂, and -NR¹⁸R¹⁹, where R¹⁸ and R¹⁹ are independently chosen from H, C₁₋₁₀ alkyl, C₇₋₁₆ aralkyl, and C₆₋₁₀ aryl. Preferably, the polar moiety is chosen from -C(=O)-R¹⁸, -C(=O)OR¹⁸, -OH, and -NR¹⁸R¹⁹, and more preferably from -C(=O)-R¹⁸, -C(=O)OR¹⁸, and -OH. Such -C(=O)-, -OH, and -NR¹⁸R¹⁹ functional groups may be part of another functional group, as in carboxylic acids, anhydrides, amides, ketones, esters, and the like. It is preferred that the polar moiety is chosen from carboxyl, C₂₋₁₂ aliphatic carboxylate, hydroxy C₁₋₁₀ alkyl, hydroxy C₆₋₁₀ aryl, C₇₋₂₀ aryl carboxylic acid, C₈₋₂₀ aryl carboxylic acid anhydride, C₇₋₂₀ aryl carboxylates, C₇₋₂₀ aryl amide, C₈₋₂₀ aryl imide, amino C₁₋₁₀ alkyl, and C₆₋₂₀ aryl amine. More preferably, the polar moiety is chosen from carboxyl, C₂₋₁₂ aliphatic carboxylate, hydroxy C₁₋₁₀ alkyl, hydroxy C₆₋₁₀ aryl, C₇₋₁₆ aryl carboxylic acid, and C₈₋₁₆ aryl carboxylic acid anhydride. Exemplary end capping monomers are: propiolic acid; acetylene dicarboxylic acid; phenyl propiolic acid; ethynyl benzoic acid; ethynyl phthalic acid; propargyl alcohol; propargylamine; 2-butyn-1,4-diol; 2-methyl-3-butyn-2-ol; 3-butyn-1-ol; 3-butyn-2-ol; 2-butyn-1-ol; 2-butynoic acid; ethynyl phenol; xylityl propiolate; ethynyl phthalic anhydride; ethynyl phthalimide; ethynyl benzamide; 2-butyn-1,4-diol diacetate; 3-butyn-2-one; 1-ethynyl-1-cyclohexanol; 1-ethynylcyclohexylamine; 1-ethynylcyclopentanol; ethynylaniline; N-(ethynylphenyl)acetamide; 2-carbamoyl-5-ethynylbenzoic acid; ethynyl-nitrobenzene; propiolamide; N-hydroxyl-propiolamide; 2-aminobut-3-ynoic acid; and mixtures thereof. Preferred end capping monomers are: propiolic acid; acetylene dicarboxylic acid; phenyl propiolic acid; ethynyl benzoic acid; ethynyl phthalic acid; propargyl alcohol; 2-butyn-1,4-diol; 2-methyl-3-butyn-2-ol; 3-butyn-1-ol; 3-butyn-2-ol; 2-butyn-1-ol; 2-butynoic acid; ethynyl phenol; xylitylpropiolate; ethynyl phthalic anhydride; 2-butyn-1,4-diol diacetate; and mixtures thereof. Such end capping monomers are generally commercially available, or may be prepared by methods known in the art.

The polymers of the present invention are prepared by reacting one or more first monomers of formula (1), one or more second monomers having two or more cyclopentadienone moieties, and optionally one or more additional monomers, such as the optional monomers of formulae (13) and/or (14) discussed above, in a suitable organic solvent. The mole ratio of the total first monomers (that is, alkyne-containing monomers) to the total second monomers (that is, monomers containing two cyclopentadienone moieties) is from 1:1.2 to 1.95:1, preferably from 1:1.15 to 1.75:1, and more preferably from 1:1.1 to 1.2:1. When an optional third monomer is used, the molar ratio of the total first monomer to the total third monomer is from 0.1:1 to 1: 0.1, preferably from 0.25:1 to 1:0.25, more preferably from 0.3:1 to 1:0.3, yet more preferably from 0.5:1 to 1:0.5, and even more preferably from 0.4:0.6 to 0.75:0.25. When an optional end capping monomer is used, it is typically used in a total amount of from 0.05 to 0.25 moles, based on 1 mole of the second monomer, preferably from 0.075 to 0.2 moles, and more preferably from 0.09 to 0.125 moles. Suitable organic solvents useful to prepare the present oligomers are benzyl esters of C₂₋₆ alkanecarboxylic acids, dibenzyl esters of C₂₋₆ alkanedicarboxylic acids, tetrahydrofurfuryl esters of C₂₋₆ alkanecarboxylic acids, ditetrahydrofurfuryl esters of C₂₋₆ alkanedicarboxylic acids, phenethyl esters of C₂₋₆ alkanecarboxylic acids, diphenethyl esters of C₂₋₆ alkanedicarboxylic acids, aromatic ethers, carbonates, and lactones. Preferred aromatic ethers are diphenyl ether, dibenzyl ether, C₁₋₆ alkoxy-substituted benzenes and benzyl C₁₋₆ alkyl ethers, and more preferably C₁₋₄ alkoxy-substituted benzenes and benzyl C₁₋₄ alkyl ethers. Preferred organic solvents are benzyl esters of C₂₋₄ alkanecarboxylic acids, dibenzyl esters of C₂₋₄ alkanedicarboxylic acids, tetrahydrofurfuryl esters of C₂₋₄ alkanecarboxylic acids, ditetrahydrofurfuryl esters of C₂₋₄ alkanedicarboxylic acids, phenethyl esters of C₂₋₄ alkanecarboxylic acids, diphenethyl esters of C₂₋₄ alkanedicarboxylic acids, C₁₋₆ alkoxy-substituted benzenes, and benzyl C₁₋₆ alkyl ethers, more preferably benzyl esters of C₂₋₆ alkanecarboxylic acids, tetrahydrofurfuryl esters of C₂₋₆ alkanecarboxylic acids, phenethyl esters of C₂₋₆ alkanecarboxylic acids, C₁₋₄ alkoxy-substituted benzenes, benzyl C₁₋₄ alkyl ethers, dibenzyl ether, carbonates, and lactones, and yet more preferably benzyl esters of C₂₋₆ alkanecarboxylic acids, tetrahydrofurfuryl esters of C₂₋₆ alkanecarboxylic acids, C₁₋₄ alkoxy-substituted benzenes, benzyl C₁₋₄ alkyl ethers, carbonates, and lactones. Exemplary organic solvents include, without limitation, benzyl acetate, benzyl proprionate, tetrahydrofurfuryl acetate, tetrahydrofurfuryl propionate, tetrahydrofurfuryl butyrate, anisole, methylanisole, dimethylanisole, dimethoxybenzene, ethylanisole, ethoxybenzene, benzyl methyl ether, benzyl ethyl ether, and propylene carbonate, and preferably benzyl acetate, benzyl proprionate, tetrahydrofurfuryl acetate, tetrahydrofurfuryl propionate, tetrahydrofurfuryl butyrate, anisole, methylanisole, dimethylanisole, dimethoxybenzene, ethylanisole, ethoxybenzene, propylene carbonate, and gamma-butyrolactone.

The oligomers of the present invention may be prepared by combining one or more first monomers, one or more second monomers, optionally one or more third monomers, optionally one or more end capping monomers, and organic solvent, each as described above, in any order in a vessel, and heating the mixture. The second monomer may be combined with the organic solvent in a vessel, and then the first monomer and any optional additional monomers are added to the mixture. In one embodiment, the second monomer and organic solvent mixture is heated to the desired reaction temperature before the first monomer is added. The first monomer may be added over a period of time, such as from 0.25 to 46 hours, and preferably from 1 to 6 hours, to reduce exotherm formation, but is preferably added at one time. The second monomer and organic solvent mixture may be heated to the desired reaction temperature before the first monomer and any optional monomers are added. Alternatively, the second monomer, first monomer, optional third monomer, optional end capping monomer and solvent are added to a vessel, and then heated to the desired reaction temperature and held at this temperature for a period of time to provide the desired oligomer. The reaction mixture is heated at a suitable temperature, such as from 85 to 205 °C. Preferably, the mixture is heated to a temperature of 90 to 160 °C, more preferably 95 to 130 °C, and yet more preferably 100 to 130 °C. The first and second monomers may react at temperatures below those conventionally used to make polyarylene polymers by a Diels-Alder type reaction. While not wishing to be bound by theory, it is believed that the presence of the solubility enhancing moiety activates the monomer such that the Diels-Alder reaction is facilitated at a lower temperature. The reaction may be carried out under oxygen-containing atmosphere, but an inert atmosphere is preferred. Following the reaction, the resulting polymer may be isolated from the reaction mixture, diluted with appropriate solvent, or used as is for coating a surface. When a first monomer having 2 alkynyl moieties having terminal hydrogens and 1 alkynyl moiety having a terminal phenyl group is used to prepare the present polymers, heating the monomer reaction mixture at a temperature of 90 to 130 °C will provide an oligomer where substantially only the alkynyl moieties having terminal hydrogens react with the first monomer to form a linear oligomer having 1 or 2 third monomers as end caps, that is, the alkynyl moieties having the terminal phenyl group remain substantially unreacted (< 10%, and preferably < 5%, of such groups react).

The present polyarylene polymers may have any suitable molecular weight range, such as a weight average molecular weight (M_{w}) of from 500 to 250000 Da (as determined by gel permeation chromatography against polystyrene standards), preferably from 1000 to 100000 Da, and more preferably from 2000 to 50000 Da. The choice of organic solvent can be used to tailor the M_{w} of the resulting oligomer. For example, when aromatic ether solvents, such as C₁₋₆ alkoxy-substituted benzenes, are used, relatively higher M_{w} oligomers may be obtained as compared to oligomers having a relatively lower M_{w} when the same reaction is performed using a benzyl ester of a C₂₋₆ alkanecarboxylic acid as the organic solvent. The molecular weight of the present oligomers can also be controlled, even in aromatic ether solvents, by adjusting the amount of the first monomer and/or optional monomers. For example, to obtain an oligomer having a M_{w} of ≤ 35000, > 1.05 mole of the first monomer should be used for each 1 mole of the second monomer, that is, the mole ratio of total alkyne monomers (that is, total second monomers and any third monomers) to total first monomers should be ≥ 1:1.05, such as from 1:1.075 to 1:1.95. As the optional end capping monomer has a single alkynyl moiety, it can be used to control the growth of the polymer chain. Increasing the total amount of any end capping monomer in the reaction will generally provide polymers having relatively lower weight average molecular weights (M_{w}), while decreasing the total amount of any end capping monomer will provide oligomers having relatively higher M_{w}.

While not intending to be bound by theory, it is believed that the present polyarylene polymers are formed through the Diels-Alder reaction of the cyclopentadienone moieties of the second monomer with the alkynyl moieties of the first monomer and the alkynyl moieties of any optional third monomers and the alkynyl moieties of any optional end capping monomers upon heating. During such Diels-Alder reaction, a carbonyl-bridged species forms. It will be appreciated by those skilled in the art that such carbonyl-bridged species may be present in the oligomers. Upon further heating, the carbonyl bridging species will be essentially fully converted to an aromatic ring system. Due to the mole ratio of the monomers used, the present polymers contain arylene rings in the polymer backbone which are substituted with at least one solubility enhancing moiety as illustrated in the following reaction scheme, where A is the first monomer and B is the second monomer. Not wishing to be bound by theory, it is believed that no unreacted cyclopentadienone moieties remain in the present oligomers.

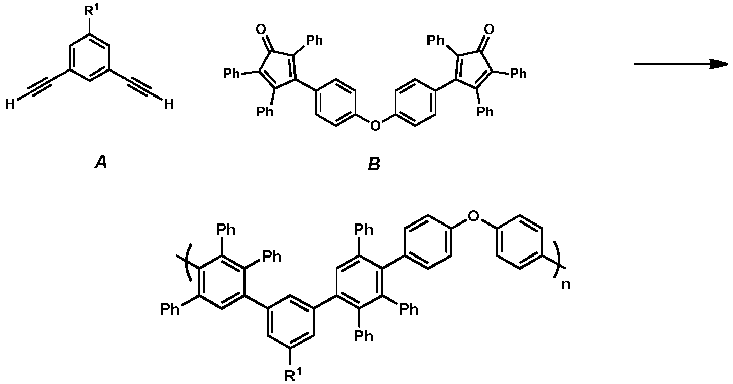

The present polymer in the organic reaction solvent can be directly cast as a film, applied as a coating or poured into a non-solvent to precipitate the oligomer or polymer. Water, methanol, ethanol and other similar polar liquids such as glycol ethers are typical nonsolvents which can be used to precipitate the oligomer. Solid oligomer may be dissolved and processed from a suitable organic solvent described above, or from organic solvents typically used in the electronics industry, such as propylene glycol methyl ether (PGME), propylene glycol methyl ether acetate (PGMEA), methyl 3-methoxypropionate (MMP), ethyl lactate, n-butyl acetate, anisole, N-methyl pyrrolidone, gamma-butyrolactone (GBL), ethoxybenzene, benzyl propionate, benzyl benzoate, propylene carbonate, and mixtures thereof. Mixtures of organic solvents are particularly preferred, such as a mixture comprising one or more of anisole, ethoxybenzene, PGME, PGMEA, GBL, MMP, n-butyl acetate, benzyl propionate and benzyl benzoate in combination with one or more additional organic solvents, and more preferably a mixture comprising two or more of anisole, ethoxybenzene, PGME, PGMEA, GBL, MMP, n-butyl acetate, benzyl propionate, and benzyl benzoate. When a mixture of solvents is used, the ratio of solvents is generally not critical and may vary from 99:1 to 1:99 w/w. The solubility enhancing moieties in the backbone of the present polymers provide improved solubility as compared to polyarylene polymers without such solubility enhancing moieties. It will be appreciated by those skilled in the art that the concentration of the polymer in the organic reaction solvent may be adjusted by removing a portion of the organic solvent, or by adding more of the organic solvent, as may be desired.

In use, the composition comprising the present polymer and organic solvent may be coated by any suitable method on any suitable substrate surface. Suitable methods for coating the composition include, but are not limited to, spin-coating, curtain coating, spray coating, roller coating, dip coating, slot die coating, and vapor deposition, among other methods. In the electronics manufacturing industry, spin-coating and slot-die coating are preferred methods to take advantage of existing equipment and processes. In spin-coating, the solids content of the composition may be adjusted, along with the spin speed, to achieve a desired thickness of the composition on the surface it is applied to. Typically, the present compositions are spin-coated at a spin speed of 400 to 4000 rpm. The amount of the composition dispensed on the wafer or substrate depends on the total solids content in the composition, the desired thickness of the resulting coating layer, and other factors well-known to those skilled in the art.

When the present oligomer compositions are used to deposit a coating or film using certain techniques, such as spin-coating, the resulting coating may suffer from certain defects. While not wishing to be bound by theory, it is believed that such defects result from the condensation of moisture on the film surface due to evaporative cooling, and such moisture forces the oligomer out of solution, resulting in a non-uniform coating of oligomer on the surface. To address such defects, a secondary solvent, which is both water-miscible and miscible with the organic solvent used in the composition, may optionally be added to the present oligomer composition. It is believed that such secondary solvent prevents the formation of water droplets during deposition of the oligomer coating on the substrate. Such secondary solvent may be added to the present composition in any suitable amount, such as from 0 to 40 wt%, based upon the total weight of the composition, and preferably from 0 to 30 wt%. Ethyl lactate and gamma-butyrolactone are examples of such a secondary solvent. Optionally, one or more secondary additives may be added to the present compositions, such as a surfactant, which may be nonionic, cationic, anionic or amphoteric. Each such secondary additive may be added to the compositions in an amount of from 0 to 5 wt%, and preferably from 0 to 2 wt%.

In general, the present compositions comprise a polyarylene oligomer of the invention, an organic solvent, and an optional secondary solvent, each as described above, wherein the oligomer is present in an amount of 1 to 35% solids, and preferably from 5 to 15% solids. Such compositions can be used to deposit an oligomer coating on a substrate, where the oligomer coating layer has a thickness of from 50 nm to 500 µm, preferably from 100 nm to 250 µm, and more preferably from 100 nm to 100 µm, although such coatings may be thicker or thinner than these ranges depending on the particular application.

Preferably, after being coated on a substrate surface, the polymer composition is heated (soft baked) to remove any organic solvent present. Typical baking temperatures are from 90 to 140 °C, although other suitable temperatures may be used. Such baking to remove residual solvent is typically done for approximately 30 sec. to 2 min., although longer or shorter times may suitably be used. Following solvent removal, a layer, film or coating of the polymer on the substrate surface is obtained. Preferably, the polymer is next cured, such as by heating to at a temperature of ≥ 300 °C, preferably ≥ 350 °C, and more preferably ≥ 400 °C. Such curing step may take from 2 to 180 min., preferably from 10 to 120 min., and more preferably from 15 to 60 min., although other suitable times may be used. In one embodiment, a belt furnace may be used to cure the polymer layer on a substrate. Upon curing, it is believed that the present polymers further polymerize. Such curing step may be performed in an oxygen-containing atmosphere, or in an inert atmosphere, and preferably in an inert atmosphere. Certain solubility enhancing moieties, such as -C(=O)OH, may be readily cleaved from the polyarylene backbone during a thermal curing step. In one preferred embodiment, the solubility enhancing moieties are substantially cleaved from the polymers during the curing step, that is ≥ 90% of the solubility enhancing moieties are cleaved, and more preferably ≥ 95% of such moieties are cleaved.

It is known that certain cured polyarylene films do not have good adhesion to substrate surfaces and require the use of an adhesion promoter, such as described in U.S. Pat. No. 5,668,210. Such adhesion promoter is typically applied to the substrate surface before the deposition of the polyarylene oligomer layer, which is subsequently cured to form the crosslinked polyarylene film. If it is desired to use an adhesion promoter, any suitable adhesion promoter for polyarylene films may be used, such as silanes, preferably organosilanes such as trimethoxyvinylsilane, triethoxyvinylsilane, hexamethyldisilazane [(CH₃)₃Si-NH-Si(CH₃)₃], or an aminosilane coupler such as gammaaminopropyltriethoxysilane, or a chelate such as aluminum monoethylacetoacetatedi-isopropylate [((i-C₃H₇O)₂Al(OCOC₂H₅CHCOCH₃))]. In some cases, the adhesion promoter is applied from 0.01 to 5 wt% solution, excess solution is removed, and then the polyarylene oligomer is applied. In other cases, for example, a chelate of aluminum monoethylacetoacetatedi-isopropylate, can be incorporated onto a substrate by spreading a toluene solution of the chelate on a substrate and then baking the coated substrate at 350 °C for 30 min. in air to form a very thin (for example 5 nm) adhesion promoting layer of aluminum oxide on the surface. Other means for depositing aluminum oxide are likewise suitable. Alternatively, the adhesion promoter, in an amount of, for example, from 0.05 to 5 wt% based on the weight of the monomer, can be blended with the monomers before polymerization, negating the need for formation of an additional layer. Particularly suitable adhesion promoters include those sold under the AP 3000, AP 8000, and AP 9000S designations, available from Dow Electronic Materials (Marlborough, Massachusetts).

The presence of solubility enhancing moieties on the polymer backbone greatly enhances the solubility of the polyarylene polymers as compared to conventional polyarylene polymers. The polymers of the present invention are particularly useful in forming a relatively low dielectric constant insulating cured polyarylene material on an electronic device substrate, such as in integrated circuits, circuit packaging applications, multichip modules, circuit boards, or displays. Cured polyarylene dielectric material produced according to the present invention may be used as is or may be combined with one or more additional dielectric materials, which may be organic or inorganic, to provide the desired insulation layer. Accordingly, the polymers of the present invention may be used to deposit a coating on a variety of electronic device substrates, including, without limitation, FR-4, silica, silicon nitride, silicon oxynitride, silicon carbide, silicon-germanium, gallium-arsenide, indium-phosphide, aluminum nitride, alumina, and the like.
Example 1: Preparation of Polymer 1. To a multineck round-bottomed flask containing a stir bar, diphenylene oxide bis(triphenylcyclopentadienone) (DPO-CPD, 15.00 g, 19.16 mmol), 3,5-diethynylbenzoic acid (DEBzOH, 1.793 g, 10.54 mmol) and 1,3,5-tris(phenylethynyl)benzene (TRIS, 3.988 g, 10.54 mmol) were added via powder funnel, followed by GBL (48 g) as the reaction solvent. The reaction was stirred gently at room temperature. The flask was next equipped with a reflux condenser and an internal thermocouple probe attached to a self-regulating thermostat control for a heating mantle. Next, the dark maroon contents of the flask were warmed to an internal temperature of 203 °C and maintained at this temperature for 60 hours before cooling to 25 °C by removal of the heating element. The resulting maroon solution was precipitated from GBL using 300 mL water heated to 70 °C as an antisolvent. Filtration and drying of the precipitate in a vacuum oven for 3 days yielded Polymer 1 as an off-white powder. Polymer 1 was analyzed by gel permeation chromatography (GPC) to provide a number-average molecular weight (Mₙ) of 6761 Da, a weight-average molecular weight (M_{w}) of 41719 Da, and a polydispersity of 6.171. This reaction is shown in Scheme 1. 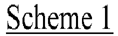 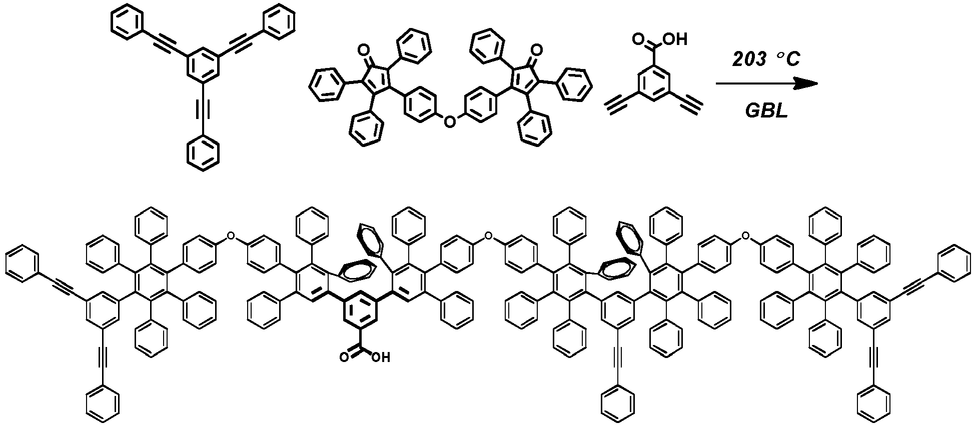 Example 2: Preparation of Polymers 2-11. The procedure of Example 1 is repeated except that 3,5-diethynylbenzoic acid is replaced with the monomers shown in Table 1.

**Table 1**

| **Polymer Number** | **Monomer** |
|---|---|
| 2 | 3,5-Bis(phenylethynyl)benzoic acid |
| 3 | Methyl 3,5-bis(phenylethynyl)benzoate |
| 4 | Methyl 3,5-diethynylbenzoate |
| 5 | Ethyl 3,5-diethynylbenzoate |
| 6 | 3,5-Bis(phenylethynyl)benzenesulfonic acid |
| 7 | N-Methyl-3,5-bis(phenylethynyl)benzamide |
| 8 | 3,5-Diethynylbenzenesulfonic acid |
| 9 | 3,5-Diethynylphenyl acetate |
| 10 | N-Methyl-3,5-diethynylbenzamide |
| 11 | N-Ethyl-3,5-diethynylbenzamide |

Example 3: Preparation of Polymers 12-15. The procedure of Example 1 is repeated except that the 1,3,5-tris(phenylethynyl)benzene is replaced with the monomers shown in Table 2.

**Table 2**

| **Polymer Number** | **Monomer** |
|---|---|
| 12 | 1,3-Diethynylbenzene |
| 13 | 1,4-Diethynylbenzene |
| 14 | 1,3-Diethynylbenzene + 1,3,5-tris(phenylethynyl)benzene (2:3 mole ratio) |
| 15 | 1,3-Diethynylbenzene + 1,3,5-tris(phenylethynyl)benzene (1:3 mole ratio) |

Example 4: Preparation of Polymers 16-22. The general procedure of Example 1 is repeated using the monomers in the molar ratios shown in Table 3.

**Table 3**

| **Polymer Number** | **Monomer** |
|---|---|
| 16 | DPO-CPD : 3,5-diethynylbenzoic acid (1:1.1) |
| 17 | DPO-CPD : 3,5-diethynylbenzoic acid : TRIS : 1,3-diethynylbenzene (1:0.5:0.35:0.25) |
| 18 | DPO-CPD : 3,5-bis(phenylethynyl)benzoic acid (1:1.15) |
| 19 | DPO-CPD : Methyl 3,5-bis(phenylethynyl)benzoate (1:1.05) |
| 20 | DPO-CPD : N-methyl-3,5-diethynylbenzamide : 1,3-diethynylbenzene (1:0.4:0:65) |
| 21 | DPO-CPD : 3,5-diethynylbenzamide : TRIS (1:0.55:0.6) |
| 22 | DPO-CPD : 3,5-diethynylbenzenesulfonic acid : 1,4-diethynylbenzene (1:0.6:0.5) |

Example 5. Polymer 23 was prepared as follows. To a three neck round-bottomed flask containing a stir bar were added DPO-CPD (25 g, 31.9 mmol), DEBzOH (1.09 g, 6.4 mmol) and GBL (88g). The reaction mixture was stirred gently at room temperature. The flask was next equipped with a reflux condenser and an internal thermocouple probe attached to a self-regulating thermostat control for a heating mantle. Next, the dark maroon contents of the flask were warmed to an internal temperature of 160 °C and maintained at this temperature for 4 hours before cooling to about 100 °C by removal of the heating element. Next, TRIS (9.66g, 25.5 mmol) was added slowly to the reaction. The resulting maroon solution was heated to 203 °C and stirred at this temperature for 47 hrs. GPC analysis of the reaction product (Polymer 23) indicated an Mₙ of 8434 Da, an M_{w} of 26395 Da, and a polydispersity of 3.13.
Example 6. The procedure of Example 5 was repeated except that the molar ratio of DPO-CPD:TRIS:DEBzOH ratio used is 1:0.9:0.2 and reaction time was 4 hours at 160 °C and 40 hours at 203 °C, to provide Polymer 24 having an Mₙ of 5486 Da.
Example 7: Solubility. A portion (5 g of 30% solids) of Polymer 23 were transferred to a transparent 20 mL vial. Ethoxybenzene (5 g) was added to the vial to make a 15% diluted Polymer 23 solution. This polymer solution was vortex mixed to ensure a uniform dispersion at room temperature. A portion (about 1 g) of this Polymer 23 solution was transferred to an empty transparent 20 mL vial. The vial was placed on a balance and an edge bead remover solvent composition based on a PGME/PGMEA solvent mixture was added dropwise into the vial using a pipette until a precipitate was visible on the vial wall or the solution becomes turbid. At this point, the amount (weight) of the solvent mixture added was noted and the ratio of the solvent mixture to polymer solution determined. If precipitation did not occur after adding 10 g of the solvent mixture, the polymer solution was considered to pass this solvent shock test. The Polymer 23 solution passed this test, and this test was repeated using Comparative Polymers 1-3. Comparative Polymer 1 was a commercially available polyarylene polymer (from Dow Electronic Materials) formed from DPO-CPD and TRIS in an approximate molar ratio of 1:1 and having an Mₙ of approximately 8800 Da. Comparative Polymer 2 was a polyarylene polymer formed from DPO-CPD and TRIS in an approximate molar ratio of 1:1 and having an Mₙ of approximately 5500 Da. Comparative Polymer 3 was prepared according to the general procedure of Example 1 of copending U.S. Pat. App. Serial No. 14/472,429 (Gilmore et al.) and formed from DPO-CPD, 1,3-diethynylbenzene and propiolic acid, and had a Mₙ of approximately 8400 Da. These results are reported in Table 4 below.
Example 8: Critical Film Thickness. Solutions of Polymer 23 as well as Comparative Polymers 1-3 were used to cast films of the respective polyarylene polymers using a slot die coater. These results are reported in Table 4, where films that passed this critical film thickness test received a score of +++, meaning there were no cracks in a film having a thickness of 1 µm. As can be seen from the data in Table 4, only polymers of the invention (Polymer 23) passed the solubility test and formed films that met or exceeded the film thickness target without cracking.

**Table 4**

| **Polymer** | **Solubility Test** | **Critical Film Thickness** |
|---|---|---|
| Polymer 23 | Pass | +++ |
| Comparative Polymer 1 | Fail | +++ |
| Comparative Polymer 2 | Pass | + |
| Comparative Polymer 3 | Fail | not tested |

Example 9. The procedure of Example 1 was repeated a number of times except that the molar ratio of DEBzOH to TRIS was varied each time. The Mₙ of each polymer was determined as described above. Each of these polymers was evaluated to determine its critical film thickness according to the procedure of Example 8 as well as its solubility according to the following test. These results are reported in Table 5.
A portion (1.2 g of) of each polymer was transferred to a transparent 20 mL vial and 8.8g of MMP/Anisole/gamma-butyrolactone (61.75/33.25/5) solvent mixture was added to make a 12% diluted polymer solution. Each polymer solution was vortex mixed to ensure a uniform dispersion at room temperature. A portion (0.6 g) of each polymer solution was added to an empty transparent 20 mL vial. The vial was placed on a balance and a conventional edge bead remover solvent composition based on a PGME/PGMEA solvent mixture having a majority of PGME was added dropwise into the vial using a pipette until a precipitate was visible on the vial wall or the solution becomes turbid. At this point, the amount (weight) of the solvent mixture added was noted and the ratio of solvent mixture to polymer solution determined. If precipitation did not occur after adding 20 g of solvent mixture, the polymer solution was considered to pass this solvent shock test. The results, reported in Table 5, clearly show the present polyarylene polymers have very good solubility in conventional edge bead remover compositions and are capable of providing films having a thickness of ≥ 1.5 µm without cracking.

**Table 5**

| **Polymer No.** | **DEBzOH: TRIS** | **Mₙ** | **Solubility Test** | **Critical Thickness** |
|---|---|---|---|---|
| 25 | 10:90 | 6400 Da | 2.3:1 | 2.5 µm |
| 26 | 20:80 | 5490 Da | 9.4:1 | 1.7 µm |
| 27 | 20:80 | 8430 Da | 2.8:1 | 2.5 µm |
| 28 | 30:70 | 6200 Da | 6.5:1 | 1.8 µm |
| 29 | 50:50 | 6760 Da | 200:1 | 2.2 µm |
| 30 | 50:50 | 5800 Da | 200:1 | 2.2 µm |
| 31 | 60:40 | 6100 Da | 100:1 | 1.8 µm |
| 32 | 60:40 | 6200 Da | 200:1 | 2.4 µm |

Example 10: Preparation of Polymer 24. The general procedure of Example 1 was repeated except that the DEBzOH monomer was replaced with methyl 3,5-diethynylbenzoate (DEBzOMe) and the molar ratio of DEBzOMe:TRIS:DPO-CPD was 0.34:0.66:1.
Example 11: Preparation of 3, 5-Diethynylphenol. 3,5-Dibromophenyl acetate was prepared as follows. The overall reaction is shown in Scheme 2 below. 3, 5-Dibromophenol (100.0 g) was added to 162.1 g of acetic anhydride at room temperature to yield a light yellow solution. The reaction was stirred at 70 °C for 6 hr. The reaction mixture was then slowly added into ice water and a white precipitate was formed. After stirring at room temperature for 0.5 hr., the solid product was collected by filtration and dried under vacuum for 12 hr. to provide 102.0 g of 3, 5-dibromophenyl acetate as a light yellow solid in 88% yield.
3, 5-Dibromophenyl acetate (50.0 g) was added to 133.0 g of 1,4-dioxane at room temperature to yield a yellow solution. Triethylamine (51.6 g) and cuprous iodide (3.23 g) were added to the reaction mixture. The reaction mixture was purged with nitrogen for 1 hr. Bis(triphenylphosphine)palladium(II) chloride (5.962 g) was added to the reaction mixture. Nest, 41.8 g of (trimethylsilyl)acetylene was slowly added to reaction mixture via an addition funnel. After completion of addition, the reaction was stirred for 24 hr. at 55 °C under nitrogen. After complete conversion, the product was filtered and solvents were evaporated. The residue was dissolved in heptanes and filtered through a silica plug. After filtration, the solvents were removed to yield 3,5-bis(trimethylsilylethynyl)phenyl acetate as a light yellow solid (51.0 g) in 91 % yield.
3,5-Bis(trimethylsilylethynyl)phenyl acetate (51.0 g) was dissolved in tetrahydrofuran (THF, 186 g) and water (46 g). The mixture was combined with 21.4 g of lithium hydroxide monohydrate at room temperature to yield a dark black reaction mixture. The reaction mixture was stirred for 6 hr. at 55 °C under nitrogen. The reaction mixture was then diluted with ethyl acetate and then treated with hydrochloric acid until the pH of aqueous layer is 4 to 5. The organic phase was separated and aqueous phase was extracted with ethyl acetate (60 mL x 3). The organic phase was washed with brine (1266 mL) and dried with MgSO₄ (10 g). The mixture was then filtered and evaporated under vacuum to give 3,5-diethynylphenol (DEPOH) as a light yellow solid (19.8 g) in 90.0% yield. 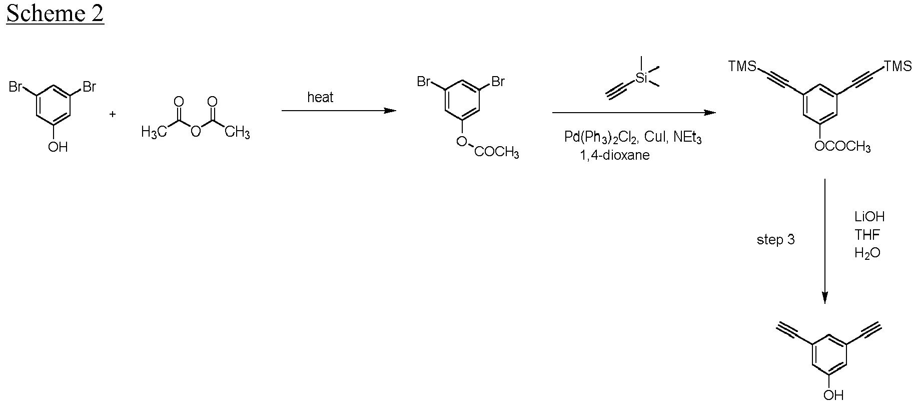 Example 12: Preparation of Polymer 33. DPO-CPD (9.0 g, 0.0115 mol) and 3,5-diethynylphenol (1.96 g, 0.0138 mol) from Example 11 were dissolved in 97 g of GBL. The reaction was heated at 120 °C for 1 hr. and then 130 °C for 1 hr. and then 150 °C for 1.5 hr. The mixture was cooled to room temperature and then diluted with 10 g of GBL. The reaction mixture was slowly added to warm water. The precipitated polymer (Polymer 33) was collected by filtration and then dried in vacuum oven at 65 to 70 deg C for 2 days. 10.0 g brown solid was obtained in 97% yield. GPC: Mw=9292, PDI=2.067. 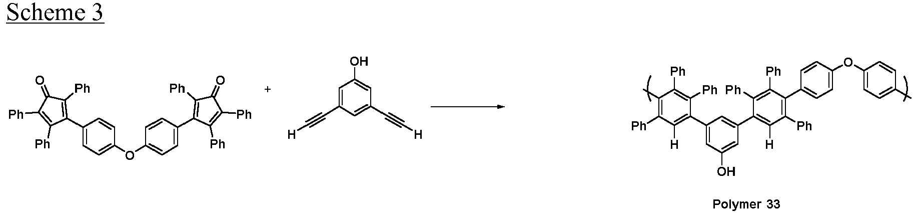 Example 13: Preparation of Polymer 34. DPO-CPD (9.0 g, 0.0115 mol) and 3,5-diethynylphenol (1.37 g, 0.0096 mol) from Example 11 were dissolved in 97 g of GBL. The reaction was heated at 130 °C for 2 hr and then cooled down to 80 °C. 1,3,5-Tris(phenylethynyl)benzene (TRIS, 1.57 g, 0.0041 mol) was then added to reaction mixture at 80 °C. The reaction was then heated at 190 °C for 16 hr. The reaction mixture was cooled to room temperature and then diluted with GBL (10 g). The reaction mixture was slowly added to warm water. The precipitated polymer was collected by filtration and then dried in vacuum oven at 65 to 70 °C for 2 days. Polymer 34 was obtained as a brown solid (10.1 g) in 98% yield. GPC: Mw=7061, PDI=3.714.
Example 14. The general procedure of Example 1 is repeated using DPO-CPD as the second monomer having two cyclopentadienone moieties, using the monomers in the mole ratios shown in Table 6 in place of the DEBzOH first monomer and TRIS third monomer. The following abbreviations are used in Table 6: 1,3-DEB = 1,3-diethynylbenzene; 1,4-DEB = 1,4-diethynylbenzene; DEPyrDOH = 4,9-diethynylpyrene-1,6-diol; DEAnDOH = 9,10-diethynylanthracene-2,6-diol; DEPerDOH = 5,8-diethynylperylene-1,12-diol; DEPyCO2H = 4,9-diethynylpyrene-1,6-dicarboxylic acid; DEBINOL = 6,6'-Diethynyl-[1,1'-binaphthalene]-2,2'-diol; DEBP = 4,4'-diethynyl-1,1'-biphenyl; and BPEBP = 4,4'-bis(phenylethynyl)-1,1'-biphenyl.

**Table 6**

| **Polymer Number** | **Monomer (mole ratio)** |
|---|---|
| 35 | DPO-CPD : DEBzOH : DEPOH (1:0.5:0.6) |
| 36 | DPO-CPD : DEBzOH : DEPOH (1:0.25:0.77) |
| 37 | DPO-CPD : DEPOH : 1,4-DEB (1:0.75:0.35) |
| 38 | DPO-CPD : Methyl 3,5-bis(phenylethynyl)benzoate : DEAnDOH (1:0.35:0.7) |
| 39 | DPO-CPD : DEPyDOH : 1,3-DEB (1:0.5:0:55) |
| 40 | DPO-CPD : DEPyDOH : TRIS (1:0.6:0.55) |
| 41 | DPO-CPD : DEPyDOH : 1,4-DEB (1:0.65:0.45) |
| 42 | DPO-CPD : DEPerDOH : TRIS (1:0.75:0.26) |
| 43 | DPO-CPD : DEPerDOH : 1,3-DEB (1:0.6:0.45) |
| 44 | DPO-CPD : DEPyCO2H : DEPOH : 1,3-DEB (1:0.3:0.45:0.27) |
| 45 | DPO-CPD : DEBP: TRIS: (1:0.75:0.26) |
| 46 | DPO-CPD : DEBOH: TRIS: (1:0.75:0.26) |
| 47 | DPO-CPD : DEBINOL : TRIS (1:0.70:0.36) |
| 48 | DPO-CPD : DEBINOL : 1,3-DEB (1:0.77:0.2) |
| 49 | DPO-CPD : DEPOH : BPEBP (1:0.65:0.47) |
| 50 | DPO-CPD : DEBzOH : DEBP (1:0.6:0.5) |

Example 15: Preparation of 6,6'-Diethynyl-[1,1'-binaphthalene]-2,2'-diol. 2,2'-Dihydroxy-6,6'-dibromo-1,1'-binaphthalene (40 g) was added to 55.1 g of acetic anhydride and 42.7 g pyridine in 250 mL dichloromethane to yield a light yellow solution. The reaction mixture was stirred at room temperature for 24 hr. The product was washed with water (3x250 mL) and the organic layer was dried over anhydrous MgSO₄. Removal of organic solvents gave 6,6'-dibromo-2,2'-diacetyl-1,1'-binaphthalene as the desired product as light yellow solid 44.0 g in 93% yield.

6,6'-Dibromo-2,2'-diacetyl-1,1'-binaphthalene (9.4 g) was added to 26.1 g of 1,4-dioxane at room temperature to yield a yellow solution. Triethylamine (5.6 g) and cuprous iodide (0.35 g) was added to the reaction mixture. The reaction mixture was purged with nitrogen for 1 hr. Bis(triphenylphosphine)palladium(II) chloride (0.975 g) was added to the reaction mixture. Trimethylsilylacetylene (5.5 g) was then slowly added to reaction mixture by way of an addition funnel. After completion of addition, the reaction was stirred for 24 hr. at 55 °C under nitrogen. After complete conversion, the product was filtered and solvents were evaporated. The residue was dissolved in heptanes and filtered through a silica plug. After filtration, the solvents were removed to yield a light yellow solid which was used in next step.

The product from previous step was dissolved in 36.4 g of THF and 8.96 g of water. To the mixture was added 4.67 g of lithium hydroxide monohydrate at room temperature to yield a dark black solution. The reaction was stirred for 24 hr. at 55 °C under nitrogen. The reaction mixture was diluted with ethyl acetate and then treated with hydrochloric acid until the pH of aqueous layer was 4 to 5. The organic phase was separated and aqueous phase was extracted with ethyl acetate (30 mL x 3). The organic phase was then washed with brine and dried with MgSO₄. The mixture was then filtered and evaporated under vacuum to give 6,6'-diethynyl-[1,1'-binaphthalene]-2,2'-diol as a light yellow solid 4.97 g in 83% yield.

Preferred Embodiments of the Present Invention are as Numbered Below:
1. A polyarylene polymer comprising as polymerized units:
   one or more first monomers of formula (1) 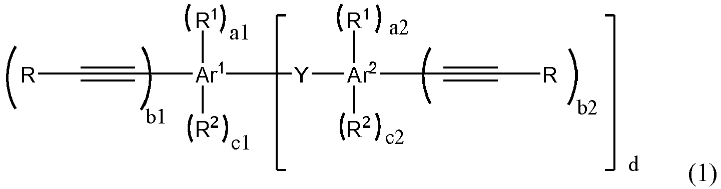 wherein each Ar¹ and Ar² is independently a C₆₋₃₀ aryl moiety; each R is independently chosen from H, C₆₋₃₀ aryl, and substituted C₆₋₃₀ aryl; each R¹ is independently chosen from -OH, C₁₋₆ hydroxyalkyl, -C(=O)OR³, -C(=O)N(R⁴)₂, -O-C(=O)R⁵, -NR⁴C(=O)R⁶, -N(R⁴)₃⁺ An⁻,-NO₂; -S(=O)₂-OR⁷, -O-S(=O)₂-R⁸, -NR⁴-S(=O)₂-R⁶, and S(=O)₂-N(R⁴)₂; each R² is independently chosen from C₁₋₁₀ alkyl, C₁₋₁₀ haloalkyl, C₁₋₁₀ hydroxyalkyl, C₁₋₁₀ alkoxy, CN, N(R⁴)₂, and halo R³ = H, C₁₋₁₀ alkyl, C₁₋₁₀ hydroxyalkyl, C₁₋₁₀ aminoalkyl, C₆₋₃₀ aryl, or M; each R⁴ is independently H, C₆₋₃₀ aryl or C₁₋₁₀ alkyl; each R⁵ is independently chosen from H, C₁₋₁₀ alkyl, C₁₋₁₀ hydroxyalkyl, C₆₋₃₀ aryl, -O(C₁₋₁₀ alkyl), -O(C₆₋₁₀ aryl) and -N(R⁴)₂; R⁶ = H, C₁₋₁₀ alkyl, C₁₋₁₀ hydroxyalkyl, C₆₋₃₀ aryl, -O(C₁₋₁₀ alkyl), or -NH(C₁₋₁₀ alkyl); R⁷ = H, C₁₋₁₀ alkyl, C₆₋₃₀ aryl, or M; R⁸ = C₆₋₃₀ aryl, C₁₋₁₀ alkyl, and halo C₁₋₁₀ alkyl; M = an alkali metal ion, an alkaline earth metal ion, or an ammonium ion; An⁻ is an anion chosen from halide and C₁₋₂₀ carboxylate; Y is a chemical bond or a divalent linking group chosen from -O-, -S-,-S(=O)-, -S(=O)₂-, -C(=O)-, -(C(R⁹)₂)_{z}-, C₆₋₃₀ aryl, and -(C(R⁹)₂)_{z1}-(C₆₋₃₀ aryl)-(C(R⁹)₂)_{z2}-; each R⁹ is independently chosen from H, hydroxy, halo, C₁₋₁₀ alkyl, C₁₋₁₀ haloalkyl, and C₆₋₃₀ aryl; a1 = 0 to 3; a2 = 0 to 3; b1 = 1 to 4; b2 = 0 to 2; c1 = 0 to 2; c2 = 0 to 2; a1 + a2 = 1 to 6; b1 + b2 = 2 to 6; c1 + c2 = 1 to 6; d = 0 to 2; z = 1 to 10; z1 = 0 to 10; z2 = 0 to 10; and z1 + z2 = 1 to 10; and
   one or more second monomers comprising two cyclopentadienone moieties.
2. The polyarylene polymer of 1 further comprising as polymerized units one or more third monomers of formula (13) 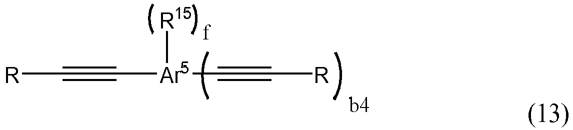 wherein Ar⁵ is a C₆₋₃₀ aromatic moiety; each R¹⁵ is independently chosen from C₁₋₄ alkyl, C₁₋₄ haloalkyl, C₁₋₄ alkoxy, optionally substituted C₇₋₁₄ aralkyl, and optionally substituted C₆₋₁₀ aryl; b4 = 1 or 2; and f = 0 to 4.
3. The polyarylene polymer of 2 wherein R¹⁵ is phenyl.
4. The polyarylene polymer of 1 wherein R¹ is chosen from -OH, -C(=O)OR³,-C(=O)N(R⁴)₂, -O-C(=O)R⁵,-S(=O)₂-OR⁵, and S(=O)₂-N(R⁴)₂.
5. The polyarylene polymer of 4 wherein R³ = H, C₁₋₆ alkyl, C₁₋₆ hydroxyalkyl, or M; R⁴ = H, or C₁₋₆ alkyl; R⁵ = H, C₁₋₆ alkyl, -O(C₁₋₆ alkyl), or -NH(C₁₋₆ alkyl); and R⁷ = H or C₁₋₆ alkyl.
6. The polyarylene polymer of 4 wherein R¹ is chosen from -OH and -C(=O)OR³.
7. The polyarylene polymer of 1 wherein R is H or phenyl.
8. The polyarylene polymer of 1 wherein the one or more first monomers have the formula (2): 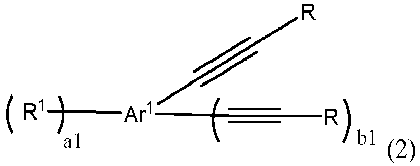 wherein Ar¹, R, R¹, a1 and b1 are as defined in 1.
9. The polyphenylene polymer of 1 wherein Ar¹ and Ar² are independently chosen from phenyl, naphthyl, anthracenyl, phenanthryl, pyrenyl, coronenyl, tetracenyl, pentacenyl, triphenylenyl, and perylenyl.
10. The polyarylene polymer of 1 wherein the one or more second monomers are chosen from one or more monomers of formula (9) 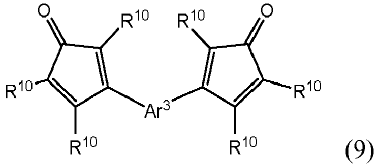 wherein each R¹⁰ is independently chosen from H, phenyl, or substituted phenyl; and Ar³ is an aromatic moiety.
11. A composition comprising one or more polyarylene polymers of 1 and one or more organic solvents.
12. The composition of 11 wherein the one or more organic solvents are chosen from propylene glycol methyl ether, propylene glycol methyl ether acetate, methyl 3-methoxypropionate, ethyl lactate, n-butyl acetate, anisole, N-methyl pyrrolidone, gamma-butyrolactone, ethoxybenzene, benzyl propionate, benzyl benzoate, propylene carbonate, and mixtures thereof.
13. A method of forming a dielectric material layer comprising: disposing a layer of the composition of 11 on a substrate surface; removing the organic solvent; and curing the oligomer to form a dielectric material layer.

## Claims

1. A polyarylene polymer comprising as polymerized units:
one or more first monomers of formula (1) 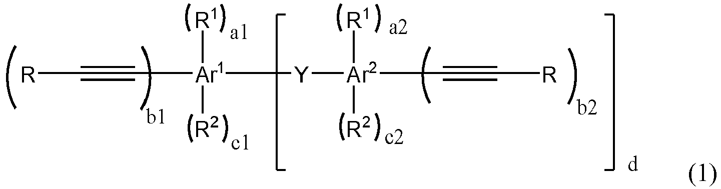 wherein each Ar¹ and Ar² is independently a C₆₋₃₀ aryl moiety; each R is independently chosen from H, C₆₋₃₀ aryl, and substituted C₆₋₃₀ aryl; each R¹ is independently chosen from -OH, C₁₋₆ hydroxyalkyl, -C(=O)OR³, -C(=O)N(R⁴)₂, -O-C(=O)R⁵, -NR⁴C(=O)R⁶, -N(R⁴)₃⁺ An⁻, -NO₂; -S(=O)₂-OR⁷, -O-S(=O)₂-R⁸, -NR⁴-S(=O)₂-R⁶, and S(=O)₂-N(R⁴)₂; each R² is independently chosen from C₁₋₁₀ alkyl, C₁₋₁₀ haloalkyl, C₁₋₁₀ hydroxyalkyl, C₁₋₁₀ alkoxy, CN, N(R⁴)₂, and halo; R³ = H, C₁₋₁₀ alkyl, C₁₋₁₀ hydroxyalkyl, C₁₋₁₀ aminoalkyl, C₆₋₃₀ aryl, or M; each R⁴ is independently H, C₆₋₃₀ aryl or C₁₋₁₀ alkyl; each R⁵ is independently chosen from H, C₁₋₁₀ alkyl, C₁₋₁₀ hydroxyalkyl, C₆₋₃₀ aryl, -O(C₁₋₁₀ alkyl), -O(C₆₋₁₀ aryl) and-N(R⁴)₂; R⁶ = H, C₁₋₁₀ alkyl, C₁₋₁₀ hydroxyalkyl, C₆₋₃₀ aryl, -O(C₁₋₁₀ alkyl), or -NH(C₁₋₁₀ alkyl); R⁷ = H, C₁₋₁₀ alkyl, C₆₋₃₀ aryl, or M; R⁸ = C₆₋₃₀ aryl, C₁₋₁₀ alkyl, and halo C₁₋₁₀ alkyl; M = an alkali metal ion, an alkaline earth metal ion, or an ammonium ion; An⁻ is an anion chosen from halide and C₁₋₂₀ carboxylate; Y is a chemical bond or a divalent linking group chosen from -O-, -S-, -S(=O)-, -S(=O)₂-, -C(=O)-, -(C(R⁹)₂)_{z}-, C₆₋₃₀ aryl, and -(C(R⁹)₂)_{z1}-(C₆₋₃₀ aryl)-(C(R⁹)₂)_{z2}-; each R⁹ is independently chosen from H, hydroxy, halo, C₁₋₁₀ alkyl, C₁₋₁₀ haloalkyl, and C₆₋₃₀ aryl; a1 = 0 to 3; a2 = 0 to 3; b1 = 1 to 4; b2 = 0 to 2; c1 = 0 to 2; c2 = 0 to 2; a1 + a2 = 1 to 6; b1 + b2 = 2 to 6; c1 + c2 = 1 to 4; d = 0 to 2; z = 1 to 10; z1 = 0 to 10; z2 = 0 to 10; and z1 + z2 = 1 to 10; with the proviso that said one or more first monomers of formula (1) is not 3,5-diethynylbenzoic acid; and
one or more second monomers comprising two cyclopentadienone moieties;
wherein the mole ratio of said one or more first monomers to said one or more second monomers is from 1:1.2 to 1.95:1.

2. The polyarylene polymer of claim 1 further comprising as polymerized units one or more third monomers of formula (13) 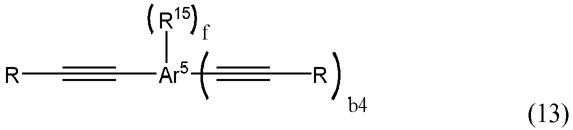 wherein Ar⁵ is a C₆₋₃₀ aromatic moiety; each R¹⁵ is independently chosen from C₁₋₄ alkyl, C₁₋₄ haloalkyl, C₁₋₄ alkoxy, optionally substituted C₇₋₁₄ aralkyl, and optionally substituted C₆₋₁₀ aryl; b4 = 1 or 2; and f = 0 to 4.

3. The polyarylene polymer of claim 2 wherein R¹⁵ is phenyl.

4. The polyarylene polymer of claim 1 wherein R¹ is chosen from -OH, -C(=O)OR³,-C(=O)N(R⁴)₂, -O-C(=O)R⁵,-S(=O)₂-OR⁵, and S(=O)₂-N(R⁴)₂.

5. The polyarylene polymer of claim 4 wherein R³ = H, C₁₋₆ alkyl, C₁₋₆ hydroxyalkyl, or M; R⁴ = H, or C₁₋₆ alkyl; R⁵ = H, C₁₋₆ alkyl, -O(C₁₋₆ alkyl), or -NH(C₁₋₆ alkyl); and R⁷ = H or C₁₋₆ alkyl.

6. The polyarylene polymer of claim 4 wherein R¹ is chosen from -OH and - C(=O)OR³.

7. The polyarylene polymer of claim 1 wherein R is H or phenyl.

8. The polyarylene polymer of claim 1 wherein the one or more first monomers have the formula (2): 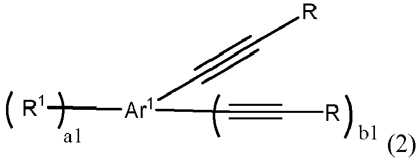 wherein Ar¹, R, R¹, a1 and b1 are as defined in claim 1.

9. The polyarylene polymer of claim 1 wherein Ar¹ and Ar² are independently chosen from phenyl, naphthyl, anthracenyl, phenanthryl, pyrenyl, coronenyl, tetracenyl, pentacenyl, triphenylenyl, and perylenyl.

10. The polyarylene polymer of claim 1 wherein the one or more second monomers are chosen from one or more monomers of formula (9) 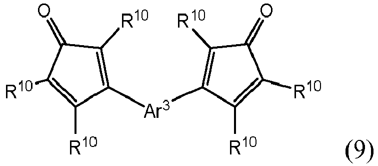 wherein each R¹⁰ is independently chosen from H, phenyl, or substituted phenyl; and Ar³ is an aromatic moiety.

11. The polyarylene polymer of claim 1 wherein R³ = H, C₁₋₄ alkyl, C₁₋₆ hydroxyalkyl, or M.

12. A composition comprising one or more polyarylene polymers of claim 1 and one or more organic solvents.

13. The composition of claim 12 wherein the one or more organic solvents are chosen from propylene glycol methyl ether, propylene glycol methyl ether acetate, methyl 3-methoxypropionate, ethyl lactate, n-butyl acetate, anisole, N-methyl pyrrolidone, gamma-butyrolactone, ethoxybenzene, benzyl propionate, benzyl benzoate, propylene carbonate, and mixtures thereof.

14. A method of forming a dielectric material layer comprising: disposing a layer of the composition of claim 12 on a substrate surface; removing the organic solvent; and curing the layer of the one or more polyarylene polymers to form a dielectric material layer.
